# FASCICULE DE BREVET EUROPEEN

(11) **EP 1 703 889 B1**
(45) Date de publication et mention de la délivrance du brevet: **23.09.2009**
(21) Numéro de dépôt: 04816412.3
(22) Date de dépôt: 17.12.2004
(51) Int. Cl.: A61K 8/97, A61K 8/36, A61K 8/34, A61Q 19/00, A61Q 19/08

(54) **UTILISATION COSMETIQUE OU PHARMACEUTIQUE D'UN EXTRAIT DE LIEGE HYDROLYSE**
KOSMETISCHE ODER PHARMAZEUTISCHE VERWENDUNG EINES KORK-HYDROLYSEEXTRAKTS
COSMETIC OR PHARMACEUTICAL USE OF A CORK HYDROLYSIS EXTRACT

(30) Priorité: 18.12.2003 FR 0314854
(43) Date de publication de la demande: 27.09.2006
(73) Titulaire: Société d'Extraction des Principes Actifs SA (Vincience), 06904 Sophis Antipolis (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2004/003277
(87) Numéro de publication internationale: WO 2005/060930

(56) Documents cités:
- EP-A- 0 974 338
- DE-C- 911 494
- FR-A- 2 841 134

## Description

L'invention concerne le domaine de la cosmétique et de la pharmaceutique, notamment le domaine de la dermatologie. La présente invention a pour objet l'utilisation, en tant qu'agent actif, d'un extrait de liège hydrolysé, dans ou pour la préparation d'une composition pharmaceutique et/ou dermatologique et/ou cosmétique.

Le liège est un tissu de revêtement secondaire que l'on rencontre chez les végétaux, notamment chez des gymnospermes et des plantes à fleurs dicotylédones. Il est constitué de cellules mortes qui forment un manchon protecteur imperméable autour des racines et des tiges des végétaux.

Le liège existe en quantité limitée dans de nombreuses espèces, mais une seule essence fournit une quantité telle qu'elle justifie le nom qu'elle porte, *Quercus suber,* le chêne-liège (Font Quer, 1977). Le liège est, en effet, présent en quantité importante dans son écorce.

Le chêne-liège, *Quercus suber,* est un arbre de la famille des Fagacées. C'est un arbre d'origine méditerranéenne, son aire de répartition naturelle est limitée à l'ouest du bassin méditerranéen. Il peut atteindre 10 à 12 mètres en France, 15 à 20 mètres en Afrique du Nord et au Portugal. Sa durée de vie moyenne est de 100 à 200 ans mais peut atteindre 300 ans si on ne l'écorce pas. Son feuillage est persistant, composé de petites feuilles dures, luisantes sur le dessus, blanchâtres, duveteuses en dessous, se renouvelant partiellement tous les 2 ou 3 ans. Le chêne-liège a une racine pivotante qui se développe en profondeur ainsi que de nombreuses racines latérales. C'est une espèce aux besoins très précis. Il ne s'établit que sur des terrains siliceux, acides et non calcaires mais s'accommode très bien de terrains pauvres. Ce chêne, *Quercus suber,* est cultivé pour sa production abondante de liège. C'est en effet la seule espèce capable de fournir une production durable et bon marché de liège. Le liège que l'arbre produit naturellement est appelé liège mâle, liège vierge ou liège naturel. Il se forme et se développe avec l'arbre, au niveau de la couche supérieure de l'écorce. Si on dépouille l'arbre de son liège mâle, par « démasclage », il se formera sous la partie mise à nu une nouvelle couche de liège désignée sous le nom de liège femelle. La récolte du liège femelle, le « levage », s'effectue tous les 9 à 12 ans, en moyenne, après le démasclage.

Le liège, ou suber, est mis en place par la face externe du méristème secondaire appelé phellogène ou assise subéreuse (Robbins, Weier et Stocking, 1958). Au cours de leur différenciation, les cellules produites sur la face externe du phellogène stockent, au niveau de leur paroi, de la subérine. Ce composé rend la paroi imperméable à l'eau et aux gaz. Les échanges avec le milieu devenant impossibles, les cellules perdent leur contenu cytoplasmique, se remplissent d'air et meurent. Composé de cellules mortes, le liège est donc un tissu étanche et très léger.

Le liège est constitué de différents composés : de subérine (45 %), de lignine (27 %), de polysaccharides (12 %), de tanins (6 %), decéroïdes (5 %) ainsi que d'eau, de glycérine et divers minéraux. La subérine est donc un de ces constituants primordial, c'est notamment elle qui confère au liège ses propriétés particulières. La subérine est une macromolécule hydrophobe, c'est un polyester de structure complexe, constitué d'acides gras hydroxylés etépoxydés. Elle contient une grande quantité d'acides gras- hydroxylés, d'acides dicarboxyliques, d'acides et d'alcools relativement longs ( > C20), ainsi qu'une petite part de monomère fortement oxydé.

De tout temps, les hommes ont utilisé le liège à des fins très diverses, notamment, depuis l'antiquité, au niveau de la pêche, de la production de chaussures ou encore dans le bouchage des liquides. Aujourd'hui, le liège a, outre son utilisation bien connue pour les bouchons de bouteille, diverses autres fonctions : isolant thermique et phonique, filtres de cigarettes, joints d'étanchéité, revêtements de sol, semelles de chaussures, etc. (Fuller, 1961). Cependant, jamais le liège n'a été utilisé pour ses propriétés biologiques et/ou pour ses vertus thérapeutiques.

Or, les inventeurs ont constaté, de manière surprenante, que des extraits de liège, et plus particulièrement des extraits de liège hydrolysés, présentent des propriétés biologiques remarquables qui les rendent directement utilisables dans des compositions pharmaceutiques, dermatologiques ou encore cosmétiques. Les inventeurs ont notamment mis en évidence les propriétés remarquables de cet extrait lorsqu'il est appliqué sur la peau et/ou sur les phanères.

Ainsi, l'invention a pour premier objet une composition cosmétique et/ou dermatologique et/ou pharmaceutique, contenant, dans un milieu cosmétiquement ou dermatologiquement acceptable, au moins un extrait de liège hydrolysé, en tant qu'agent actif, seul ou en association avec au moins un autre agent actif, et l'extrait de liège contenant majoritairement des alcools et des acides gras.

L'invention a pour second objet une utilisation d'une quantité efficace d'au moins un extrait de liège hydrolysé, en tant qu'agent actif, seul ou en association avec au moins un autre agent actif, dans ou pour la préparation d'une composition pharmaceutique et/ou dermatologique et/ou cosmétique, ledit extrait de liège contenant majoritairement des alcools et des acides gras.

L'invention a également pour objet un procédé de traitement cosmétique de la peau destiné à obtenir un lissage mécanique et immédiat de la surface de la peau, consistant à appliquer, sur la peau, une composition cosmétique selon l'invention.

Du fait de leurs multiples propriétés décrites plus en détail ci-après, ces extraits trouveront avantageusement des utilisations, seuls ou en associations, avec d'autres composés actifs, dans des produits destinés à être appliqués sur la peau et/ou sur les phanères ; et plus particulièrement, dans des produits destinés à être utilisés afin de lutter contre le vieillissement de la peau ou contre la perte d'élasticité de celle-ci.

Le principe actif peut se définir comme étant une molécule ou un ensemble de molécules susceptibles d'apporter des modifications ou des modulations au fonctionnement d'un système biologique.

Le terme « extrait » désigne tout produit ou préparation obtenu à partir de substance végétale (ou animale) sèche. Il s'obtient, par exemple, par dissolution des actifs au moyen de solvants (comme l'eau, l'alcool ou encore l'éther) puis par concentration de ces actifs, par exemple, par évaporation des solvants. Par « extrait de liège », on entend toutes substances isolées, obtenues à partir de la matière première végétale que constitue le liège. Cet extrait contiendra le maximum de composés extractibles. Tous ces composés seront obtenus à partir de la matière première végétale que constitue le liège.

L'extrait de liège selon l'invention, obtenu par une méthode classique d'extraction, bien connue de l'homme du métier, permettra d'obtenir une composition contenant différents types de molécules que l'on retrouve dans le liège. Ces molécules pourront être, par exemple, des acides gras solubles, des tripterpènes, des tanins, des céroïdes... Selon un mode de réalisation préféré de l'invention, l'extrait est préparé à partir de liège provenant du chêne-liège, *Quercus suber.*

L'expression « extrait de liège hydrolysé », désigne un extrait de liège qui a subi une étape d'hydrolyse. L'hydrolyse se définit comme étant une réaction chimique impliquant le clivage d'une molécule par de l'eau, cette réaction pouvant se faire en milieu neutre, acide ou basique.

L'hydrolyse de l'extrait de liège selon l'invention permet ici le clivage des macromolécules et des différents polymères que l'on retrouve dans le liège. Il permet notamment de cliver, en composés de plus petites tailles, des macromolécules telles que la subérine. L'hydrolyse de l'extrait de liège s'effectuera, principalement, au niveau des liaisons esters qui forment le polymère de subérine.

Selon un mode de réalisation avantageux de l'invention, l'hydrolyse de l'extrait de liège se fait en milieu basique, par une réaction de saponification. On obtient ainsi un extrait de liège saponifié, c'est-à-dire un extrait qui a subi une étape de saponification.

La réaction de saponification se définit comme étant une hydrolyse des esters, en présence d'une base (comme par exemple la soude ou la potasse), qui libère une fraction acide et une fraction alcoolique.

L'extrait de liège ainsi hydrolysé selon l'invention aura une composition proche de celle de la subérine ; c'est à dire que l'on retrouvera les différents composés majeurs qui la constitue. Ainsi, l'extrait de liège saponifié ou hydrolysé, selon l'invention, contiendra majoritairement des alcools et des acides gras. Plus précisément, l'extrait de liège hydrolysé selon la présente invention contiendra, par exemple, des acides ω-hydroxylés (en C₁₆-C₃₂), des acides octadecanoïques, des acides octadecane-1,18-dioïque, des diacides gras ω (en C₁₆-C₃₂), des acides gras (en C₁₆-C₃₂), des alcools gras primaire (en C₁₈-C₃₂), mais aussi divers composés phénoliques comprenant, entre autres, de l'acide férulique (4-hydroxy-3-méthoxycinnamicacide), ou encore de l'acide caféique (hydroxycinnamicacide). La composition de l'extrait de liège saponifié sera très proche de la composition de la subérine, proche à environ 95 %.

Plus précisément, l'extrait de liège saponifié, selon l'invention contiendra entre 35 et 45 % d'acides ω-hydroxylés (en C₁₆-C₃₂), entre 18 et 25 % d'acides octadecanoïques, entre 20 et 25 % d'acides octadecane-1,18-dioïque, entre 7 et 10 % de diacides gras ω (en C₁₆-C₃₂), entre 1 et 5 % d'alcools gras primaire (en C₁₈-C₃₂), et entre 1 et 3 % d'acides gras (en C₁₆-C₃₂).

Préférentiellement selon l'invention, le principe actif est obtenu par extraction et/ou purification à partir de la matière première naturelle végétale que constitue le liège. Toute méthode d'extraction ou de purification, ainsi que toute méthode d'hydrolyse, connue de l'homme du métier peut être utilisée pour préparer l'extrait actif selon l'invention.

Dans une première étape, la matière première végétale, issue du liège brut mâle ou femelle, est lavée. Ce liège est alors réduit en une poudre de granulométrie plus ou moins fine, de préférence la granulométrie sera inférieure à 5 millimètres. La poudre de liège peut être utilisée telle qu'ainsi obtenue. Cependant, une extraction préalable peut être aussi réalisée par de l'eau ou par un solvant organique tel que l'éthanol, l'acétone, l'acétate d'éthyle, un solvant chloré ou encore un hydrocarbure. Selon une variante du procédé de l'invention, il est donc possible d'utiliser, en tant que matière première, un extrait de liège afin de préparer un extrait hydrolysé de liège.

L'hydrolyse proprement dite est réalisée, selon les méthodes classiques d'hydrolyse, dans un milieu aqueux ou à l'aide d'un solvant, à température et à pression ambiante, ou, selon encore une autre méthode, à une température élevée et sous pression.

Selon une variante du procédé d'obtention de l'extrait hydrolysé de liège selon l'invention, l'hydrolyse peut être effectuée par trans-estérification en présence d'un alcoolate tel que le méthylate de sodium, l'éthylate de sodium, un acide, un acide de Lewis ou tout autre produit classiquement utilisé à cet effet.

Ce procédé nous permet ainsi d'obtenir l'ester de l'alcool mis en oeuvre tel que, par exemple, un ester méthylique ou un ester éthylique. Ces esters sont, par la suite, hydrolysés dans le but d'obtenir des produits sous forme acide ou bien sous forme de sels, c'est-à-dire par réaction avec une base ou avec un acide en milieu aqueux ou avec un solvant. Selon une méthode actuellement préférée de réalisation de l'invention, une réaction de saponification est réalisée : c'est-à-dire que l'hydrolyse est effectuée par une base forte en milieu aqueux ou dans un solvant.

Une fois la réaction de saponification effectuée, le milieu réactionnel est filtré afin d'éliminer, notamment, les constituants du liège qui n'auraient pas été solubilisés. Le milieu ainsi clarifié est neutralisé, les extractibles sont récupérés par décantation et filtration, ou avec l'aide d'un solvant non miscible à l'eau, ou encore, après évaporation. Les produits ainsi extraits se présentent sous la forme d'un acide ou bien sous forme d'un sel.

Selon un mode de réalisation avantageux de l'invention, l'extrait de liège hydrolysé précité est préalablement solubilisé dans un ou plusieurs solvants cosmétiquement ou pharmaceutiquement acceptables, classiquement utilisés par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, l'extrait précité est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu évident que l'extrait selon l'invention peut être utilisé seul ou bien en association avec au moins un autre agent actif, dans ou pour la préparation d'une composition pharmaceutique et/ou dermatologique et/ou cosmétique.

L'invention a, en outre, pour objet l'utilisation d'un extrait de liège hydrolysé, tel que défini précédemment, dans ou pour la préparation d'une composition ; l'agent actif ou la composition étant destinés à traiter de manière curative et/ou préventive les manifestations cutanées du vieillissement, et/ou à améliorer l'aspect de la peau et/ou des phanères. Plus généralement l'extrait selon l'invention aura une activité efficace dans tous les soins de la peau et/ou des phanères.

Par les soins de la peau et/ou des phanères, on entend toutes les actions destinées à conserver ou à rétablir un bon fonctionnement de la peau et/ou des phanères ou encore tout moyen qui sert à préserver ou à améliorer leur apparence et/ou leur aspect. Ainsi le soin inclut l'hydratation, la protection contre tous types d'agression, notamment la protection solaire, la lutte et la prévention des manifestations du vieillissement, notamment les manifestations cutanées du vieillissement. Par manifestations cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultraviolets.

Par l'expression "améliorer l'aspect de la peau", on entend tous les phénomènes qui sont susceptibles d'avoir pour conséquence une amélioration visuelle de l'état de la peau. La peau présentera une meilleure apparence ; elle sera, par exemple, beaucoup plus belle, ferme et/ou lisse. Toutes les petites imperfections seront diminuées ou supprimées. L'aspect papyracé de la peau sera, par exemple, atténué.

Par ailleurs, l'extrait de liège hydrolysé selon l'invention possède comme propriété remarquable un effet tenseur immédiat de la peau, lorsqu'il est appliqué sur celle-ci.

Cette particularité nous permet ainsi de disposer d'un actif qui possède un effet liftant et lissant immédiat. L'extrait selon l'invention agit sur la peau de manière à provoquer une tension superficielle de celle-ci ; cette action conduit à des résultats visibles immédiatement. L'extrait de liège hydrolysé selon l'invention conduit ainsi rapidement à un lissage des rides et/ou des ridules ainsi qu'à la disparition, même temporaire, des marques de fatigue. Cet extrait agit, notamment, en formant un film provoquant la rétraction du *stratum corneum,* la couche cornée superficielle de l'épiderme. Ainsi l'invention concerne l'utilisation d'une quantité efficace d'au moins un extrait de liège hydrolysé, dans ou pour la préparation d'une composition, l'extrait ou la composition étant utilisés en tant qu'agent tenseur de la peau. On entend par « agent tenseur » des composés susceptibles d'avoir un effet tenseur, c'est-à-dire pouvant tendre la peau et, par cet effet de tension, lisser la peau et y faire diminuer, voire disparaître, de façon immédiate les rides et les ridules. Le composé selon l'invention permet de corriger les signes cliniques du vieillissement de la peau liés à un relâchement cutané, en particulier pour lisser la peau et/ou les ridules.

De plus, le principe actif ou la composition selon l'invention peuvent être destinés à protéger les substrats kératiniques, et plus particulièrement à protéger la peau et/ou les phanères contre tous les types d'agressions extérieures. L'utilisation de cet agent actif, ou d'une composition le contenant, va permettre aux substrats kératiniques d'être protégés et de mieux résister au stress que produit sur eux l'environnement. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums. Par ailleurs, l'agent actif, ou la composition le contenant, a des effets anti-inflammatoires et anti-irritants. L'utilisation des propriétés de cet agent cosmétique permet donc d'avoir une peau plus protégée et nettement moins sensible aux diverses agressions qu'elle peut rencontrer. La peau est ainsi apaisée.

De plus, l'agent actif selon l'invention stimule le fonctionnement métabolique des cellules, et notamment des cellules de la peau ; il permet ainsi d'augmenter la synthèse de protéines essentielles à son fonctionnement, notamment en augmentant la synthèse de protéines constitutives de la matrice extra-cellulaire. L'agent actif selon l'invention, ou la composition le contenant, possède ainsi une action positive sur la régénération tissulaire et sur la cicatrisation.

Selon un autre aspect, l'invention a pour objet une composition cosmétique et/ou dermatologique et/ou dermo-pharmaceutique contenant, comme principe actif, dans un milieu cosmétiquement ou pharmaceutiquement acceptable, au moins un composé tel que défini précédemment ; c'est-à-dire un extrait de liège hydrolysé.

La composition contenant l'extrait de liège hydrolysé selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable. Préférentiellement, la composition selon l'invention est adaptée à une application sur la peau.

Il est bien évident que l'invention s'adresse aux mammifères en général et plus particulièrement aux êtres humains.

La quantité efficace de principe actif correspond à la quantité nécessaire pour obtenir le résultat désiré. Ainsi, selon un mode de réalisation avantageux de l'invention, l'extrait précité est présent dans les compositions de l'invention à une quantité comprise entre 0,0001 % et 10 % en poids, préférentiellement à une quantité comprise entre 0,001 % et 5 % en poids, et d'une manière encore plus préférentielle à une quantité comprise entre 0,05 % et 3 % en poids par rapport au poids total de la composition finale.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées. Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les muqueuses, les poils ou les cheveux. Ces compositions pourront notamment se présenter sous forme d'une solution aqueuse, hydralcoolique ou huileuse ; d'une émulsion huile-dans-eau, eau-dans-huile ou émulsions multiples ; elles aussi peuvent se présenter sous forme de crèmes, de suspensions, ou encore poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif couramment utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc. Dans tous les cas, l'homme du métier veillera à ce que ces adjuvants ainsi que leurs proportions soient choisis de manière telle à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semi-muqueuses, mais aussi comme compositions cosmétiques ou pharmaceutiques pour les phanères et/ou les cheveux. Elles trouvent une application toute particulière en tant que produit de protection et/ou de soin de la peau, ou encore en tant que composition anti-rides et/ou anti-âge, notamment pour la peau du visage et/ou du cou. Tout particulièrement, les compositions trouvent une application en tant que composition destinée à avoir un effet tenseur sur la peau.

On peut également envisager une application dans le domaine des compositions de maquillage de la peau du visage et du corps, telles que les rouges à lèvres, les fond de teint, les crèmes teintées, les sticks anti-cernes, ou les compositions anti-solaires ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, par exemple une pâte de dentifrice. La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule, ou encore d'un aliment ou complément nutritionnel. Selon l'invention, on peut ajouter à la composition de l'invention d'autres agents actifs destinés, entre autres, à la prévention et/ou au traitement des manifestations cutanées du vieillissement, ou bien destinés à la protection de la peau contre les agressions extérieures.

Selon un autre aspect, la présente invention concerne un procédé de traitement cosmétique destiné à traiter les peaux âgées et/ou à combattre de manière curative et/ou préventive les phénomènes de vieillissement cutané consistant à appliquer, sur la surface de la peau, une quantité efficace d'une composition telle que définie précédemment. C'est-à-dire une composition contenant un extrait de liège hydrolysé, afin d'obtenir l'action désirée.

La présente invention concerne aussi un procédé de traitement cosmétique de la peau en vue d'obtenir un effet tenseur, c'est-à-dire un lissage mécanique et immédiat de la surface de la peau, consistant à appliquer, sur la peau, une quantité efficace d'une composition telle que définie précédemment.

La présente invention concerne, de la même manière, un procédé de traitement cosmétique afin de protéger la peau et/ou les cheveux contre tous types d'agressions extérieures.

Des modes de réalisation particuliers de ce procédé de traitement cosmétique résultent également de la description précédente.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

### Exemple 1 : Préparation de l'extrait de liège hydrolysé.

Une quantité d'un kilogramme de liège, préalablement lavé avec de l'alcool, est réduit en une poudre de granulométrie inférieure à 1 millimètre.

Ce liège subit alors une réaction de saponification par solubilisation dans 10 litres d'une solution aqueuse de potasse à 2 N. La réaction de saponification est alors effectuée à reflux durant environ 6 heures, à une température comprise entre 90 et 130°C.

Par la suite, le milieu réactionnel est refroidi puis filtré sur un filtre à plaque ou sur un filtre à poche. Le résidu solide est écarté. Le filtrat ainsi obtenu est neutralisé et amené à un pH acide. Ce résidu est alors extrait trois fois par 1 litre d'hexane, puis les fractions organiques sont réunies et évaporées.

Ces différentes étapes nous permettent d'obtenir entre 0,2 et 0,4 kg d'une « pâte » de couleur jaune orangée. C'est cette pâte qui constitue l'extrait de liège hydrolysé selon l'invention. Cette pâte sera, par la suite, mise en solution à, environ, 5 ou 10 % dans un solvant de type glycol. Cette solution constituera « l'extrait de l'exemple 1 ». Cet extrait sera ensuite formulé dans un milieu cosmétiquement ou pharmaceutiquement acceptable.

Selon une variante du procédé de préparation, l'extrait de liège hydrolysé selon l'invention peut être obtenu par une réaction de méthonolyse, c'est-à-dire par une réaction de trans-estérification.

Pour cela la poudre de liège telle qu'obtenue précédemment est traitée par 10 litres d'une solution de méthylate de sodium à 0,03 %. La réaction est effectuée, comme précédemment, à reflux pendant 6 heures. Le milieu réactionnel refroidi est ensuite filtré et le résidu solide est écarté. Puis le filtrat est évaporé et le résidu est remis en suspension dans une solution aqueuse de potasse à 1 N, celui-ci est alors chauffé à reflux durant deux heures.

Le milieu réactionnel est par la suite refroidi, puis deux volumes d'eau sont ajoutés. Le milieu réactionnel est finalement amené à un pH acide. Ce résidu est alors extrait trois fois par 1 litre d'hexane, puis les fractions organiques sont réunies et évaporées. On récupère alors entre 0,2 et 0,4 kg d'une pâte de couleur jaune orangée qui constitue l'extrait de liège hydrolysé selon l'invention.

### Exemple 2 : Mise en évidence de l'effet tenseur de l'extrait de l'exemple 1.

### 1. Principe du test

Un test in-vivo a été effectué sur des volontaires de façon à mettre en évidence l'effet tenseur de l'extrait le liège hydrolysé selon l'invention. Ce test a été réalisé par l'étude de deux paramètres : une analyse de la rugosité de la surface cutanée ainsi qu'une analyse des photos des zones de peau traitées.

### 2. Modèle expérimental

Une étude clinique a été effectuée sur un groupe de 15 volontaires, âgés de 22 à 52 ans. Cette étude a été réalisée en double aveugle contre placebo sur une durée de 2 heures.

Une seule application, à raison de 2 mg/cm², a été réalisée sur des zones de 30 cm² de peau selon une liste de randomisation, un bras recevant l'actif formulé à 3 %, dans un gel, tandis que l'autre recevait le placebo. La prise des photos et des empreintes de silicones a été effectuée à T0h, c'est-à-dire avant l'application des produits, ainsi qu'à 1 et 2 heures après l'application des produits, c'est-à-dire à T1h et T2h.

La mesure de l'effet tenseur du liège a été étudiée à l'aide de 2 paramètres:
- Les photos, réalisées au moyen de la caméra Visioscan VC 98 (Courage & Khazaka).
- La rugosité de la surface cutanée, analysée à l'aide d'empreintes de silicone (Siflo). Après l'acquisition numérique de ces empreintes, à l'aide d'une caméra CCD noire et blanche, (COHU High Performance), elles sont traitées à l'aide du logiciel Quantilines (Monaderm). Les 2 paramètres principaux de l'analyse de ces empreintes sont la rugosité moyenne (Ra) et la rugosité maximale (Rz).

### 3. Résultats

Les photos ont été analysées par une lecture en aveugle afin de déterminer la présence d'une amélioration ou non, pendant le test, aux temps T2h et T1h par rapport au temps initial T0h.

Étant donné la non-normalité de distribution des résultats des analyses de la rugosité de la surface cutanée, c'est-à-dire des scores Ra et Rz enregistrés, les analyses ont été analysées qualitativement suivant les évolutions observées. Ainsi, les résultats obtenus sur les différents individus ont été classés selon 2 paramètres : « amélioré » ou « non amélioré », c'est-à-dire suivant le fait que les différences constatées soient positives (amélioré) ou négatives (non amélioré).

Une étude statistique de cette classification, sur l'observation de la présence ou de l'absence d'un effet de l'actif en comparaison avec le placebo, a été effectuée grâce au test non paramétrique de McNemar.

Cette étude nous permet d'obtenir différents résultats :

| | **T1h** | **T2h** |
|---|---|---|
| Photos | 0.0098 ** | 0.0008 *** |
| Empreintes Ra | 0.3528 | 0.0513 * |
| Empreintes Rz | 0.3274 | 0.1284 |

| | | |
|---|---|---|
| (*) : significatif - (**) : très significatif - (***) : hautement significatif | | |

L'analyse de ces résultats nous permet de conclure qu'il y a eu une amélioration chez les volontaires qui ont appliqué l'actif, par rapport au placebo. Cette amélioration est de, respectivement, 73,3 % au temps T1h et de 78,6 % au temps T2h, ce qui représente une différence significative de 0,0098 et 0,0008.

En conclusion, il est constaté que l'extrait de liège hydrolysé selon l'invention, formulé à 3% dans un gel, possède un effet tenseur important, c'est-à-dire un lissage et une diminution des rides immédiatement après application de l'actif.

### Exemple 3 - Préparation de compositions

Ces compositions ont été obtenues par simple mélange des différents composants. Les quantités indiquées sont données en pourcentage de poids.

### 1. Emulsion huile-dans-eau

| ***Noms commerciaux*** | ***Noms INCI*** | ***% massique*** |
|---|---|---|
| ***PHASE HUILEUSE*** | | |
| Montanov 68 | Cetearyl Alcohol (and) Cetearyl Glucoside | 5.00 |
| Extrait de l'exemple 1 | | 1.00 |
| Huile de Jojoba | Simmondsia Chimensis Seed Oil | 5.00 |
| Huile de Vaseline | Paraffinum Liquidum (Mineral oil) | 5.00 |
| Isopropyl Palmitate | Isopropyl Palmitate | 7.00 |
| ***PHASE AQUEUSE*** | | |
| Glycerine | Glycerin | 5.00 |
| Allantoïne | Allantoin | 0.10 |
| Sepigel 305 | Polyacrylamide (and) C13-14 Isoparaffin (and) Laureth-7 | 0,30 |
| Conservateur | | 0,50 |
| Parfum | Parfum (Fragrance) | 0,50 |
| Eau déminéralisée | Aqua (Water) | Qsp |

### 2. Lotion

| ***Noms commerciaux*** | ***Noms INCI*** | **% *massique*** |
|---|---|---|
| Mono Propylene Glycol | Propylene Glycol | 1.00 |
| Allantoïne | Allantoin | 0.30 |
| Glycérine | Glycerin | 1.00 |
| Cetiol HE | PEG-7 Glyceryl Cocoate | 1.00 |
| Extrait de l'exemple 1 | | 0.50 |
| Conservateur | | 0.20 |
| Parfum | Parfum (Fragrance) | 0.50 |
| Eau déminéralisée | Aqua (Water) | qsp |

### 3. Gel

| ***Noms commerciaux*** | ***Noms INCI*** | **% *massique*** |
|---|---|---|
| Carbopol Ultrez 10 (2%) | Carbomer | 25.00 |
| Triéthanolamine | triethanolamine | 0,50 |
| Extrait de l'exemple 1 | | 5.00 |
| Conservateur | | 0,20 |
| EDTA | Tetrasodium EDTA | 0.10 |
| Parfum | Parfum (Fragrance) | 0,50 |
| Colorant hydrosoluble | | Qsp |
| Eau déminéralisée | Aqua (Water) | Qsp |

### 4. Sérum tenseur

| ***Noms commerciaux*** | ***Noms INCI*** | **% *massique*** |
|---|---|---|
| ***PHASE A*** | | |
| Glycerol | Glycerin | 3.00 |
| PVP K30 | PVP | 0.5 |
| Keltroll TF | Xanthan Gum | 0.3 |
| Eau déminéralisée | Aqua (Water) | qsp |
| ***PHASE B*** | | |
| Simulgel EG | Sodium Acrylate/Acryloyldimethyl Taurate Copolymer (and) Isohexadecane (and) Polysorbatè 80 | 1.00 |
| Phenonip | Phenoxyethanol (and) Methylparaben (and) Ethylparaben (and) Butylparaben (and) Propylparaben (and) Isobutylparaben | 0.50 |
| ***PHASE C*** | | |
| Extrait de l'exemple 1 | | 5.00 |
| Parfum | Parfum (Fragrance) | 0.50 |

## Revendications

1. Composition cosmétique et/ou dermatologique et/ou pharmaceutique, contenant, dans un milieu cosmétiquement ou dermatologiquement acceptable, au moins un extrait de liège hydrolysé, en tant qu'agent actif, seul ou en association avec au moins un autre agent actif, **caractérisée en ce que** l'extrait de liège comprend entre 35 et 45 % d'acides ω-hydroxylés en C16-C32, entre 18 et 25 % d'acides octadecanoïques, entre 20 et 25 % d'acides octadecane-1,18-dioïque, entre 7 et 10 % de diacides gras ω en C16-C32, entre 1 et 5 % d'alcools gras primaire en C18-C32, et entre 1 et 3 % d'acides gras en C16-C32.

2. Composition selon la revendication 1, **caractérisée en ce qu'**elle se présente sous la forme d'une composition cosmétique et/ou dermatologique adaptée à l'administration par voie topique cutanée comprenant un milieu cosmétiquement ou pharmaceutiquement acceptable.

3. Composition selon l'une des revendications précédentes, **caractérisée en ce que** L'extrait de liège hydrolysé est présent à une quantité comprise entre 0,0001 % et 10 % en poids, préférentiellement à une quantité comprise entre 0,001 % et 5 % en poids, et d'une manière encore plus préférentielle à une quantité comprise entre 0,05 % et 3 % en poids par rapport au poids total de la composition finale.

4. Composition selon l'une des revendications précédentes, **caractérisée en ce que** l'extrait est préalablement solubilisé ou dispersé dans un ou plusieurs solvants cosmétiquement ou pharmaceutiquement acceptables comme l'éthanol, le propanol ou l'isopropanol, le propylène glycol, le butylène glycol, le dipropylene glycol, les diglycols éthoxylés ou propoxylès ou tout mélange de ces solvants.

5. Composition selon l'une des revendications précédentes, **caractérisée en ce que** l'extrait est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

6. Composition selon l'une quelconque des revendications précédentes, **caractérisée en ce qu'**elle se présente sous forme d'une solution aqueuse, hydralcoolique ou huileuse ou sous la forme d'une émulsion huile-dans-eau, eau-dans-huile ou émulsions multiples ou sous forme de crèmes, de suspensions ou encore de poudres ; ces compositions pouvant être plus ou moins fluides ou solides et avoir l'aspect d'une crème, d'une lotion, d'un lait, d'un sérum, d'une pommade, d'un gel, d'une pâte, d'une mousse ou d'un stick.

7. Utilisation cosmétique d'une quantité efficace d'au moins un extrait de liège hydrolysé, en tant qu'agent actif, seul ou en association avec au moins un autre agent actif, ledit extrait de liège contenant majoritairement des alcools et des acides gras, lesdits alcools et acides gras résultant de l'hydrolyse des esters de l'extrait de liège, dans une composition, en tant qu'agent tenseur de la peau pour obtenir un lissage mécanique de la peau.

8. Utilisation selon la revendication 7 pour former un film provoquant la rétraction de la couche cornée superficielle de l'épiderme et tendre la peau.

9. Procécé de traitement cosmétique de la peau destiné à obtenir un lissage mécanique et immédiat de la surface de la peau, consistant à appliquer, sur la peau, une composition cosmétique définie selon l'une quelconque des revendications 1 à 6 ou une composition comprenant un extrait de liège tel que défini dans la revendication 7.

## Claims

1. Cosmetic and/or dermatological and/or pharmaceutical composition, containing, in a cosmetically or dermatologically acceptable medium, at least one hydrolysed cork extract, as an active ingredient, alone or in association with at least one other active agent, wherein the cork extract comprises between 35 and 45% C16-C32 ω-hydroxylated acids, between 18 and 25% octadecanoic acids, between 20 and 25% octadecane-1,18-dioic acids, between 7 and 10% C16-C32 ω fatty diacids, between 1 and 5% C18-C32 primary fatty alcohols, and between 1 and 3% C16-C32 fatty acids.

2. Composition according to claim 1, which is presented in the form of a cosmetic and/or dermatological composition suitable for cutaneous topical administration comprising a cosmetically or pharmaceutically acceptable medium.

3. Composition according to any of the above claims, wherein the hydrolysed cork extract is present at a quantity between 0.0001% and 10% by weight, preferentially at a quantity between 0.001% and 5% by weight, and more preferentially at a quantity between 0.05% and 3% by weight with respect to the total weight of the final composition.

4. Composition according to any of the above claims, wherein the extract is previously solubilised or dispersed in one or a plurality of cosmetically or pharmaceutically acceptable solvents such as ethanol, propanol or isopropanol, propylene glycol, butylene glycol, dipropylene glycol, ethoxylated or propoxylated diglycols or any mixture of said solvents.

5. Composition according to any of the above claims, wherein the extract is previously solubilised in a cosmetic or pharmaceutical vector such as liposomes or adsorbed on powdery organic polymers, mineral substrates such as talcs and bentonites, and more generally solubilised in, or fixed on, any cosmetically or pharmaceutically acceptable vector.

6. Composition according to any of the above claims, which is presented in the form of an aqueous, hydroalcoholic or oily solution or in the form of an oil-in-water emulsion, water-in-oil emulsion or multiple emulsions or in the form of creams, suspensions or powders; these compositions possibly being more or less fluid or solid and having the appearance of a cream, lotion, milk, serum, ointment, gel, paste, foam or stick.

7. Cosmetic use of an effective quantity of at least one hydrolysed cork extract, as an active agent, alone or in association with at least one other active agent, said cork extract mainly containing alcohols and fatty acids, said alcohols and fatty acids resulting from the hydrolysis of the esters of the cork extract in a composition, as a skin tensor agent to obtain mechanical smoothing of the skin.

8. Use according to claim 7 to form a film inducing the retraction of the superficial stratum corneum epidermidis and to stretch the skin.

9. Method for the cosmetic treatment of the skin to obtain immediate mechanical smoothing of the skin surface, consisting of applying, on the skin, a cosmetic composition defined according to any of claims 1 to 6 or a composition comprising a cork extract as defined in claim 7.

## Patentansprüche

1. Kosmetische und/oder dermatologische und/oder pharmazeutische Zusammensetzung, die in einem kosmetisch oder dermatologisch akzeptablen Milieu mindestens einen hydrolysierten Korkextrakt als Wirkstoff enthält, allein oder in Verbindung mit mindestens einem anderen Wirkstoff, **dadurch gekennzeichnet, dass** der Korkextrakt zwischen 35 und 45 % ω-hydroxylierte C16-C32-Säuren, zwischen 18 und 25 % Octadecansäuren, zwischen 20 und 25 % Octadecan-1,18-diolsäuren, zwischen 7 und 10 % zweibasige C16-C32-ω-Fettsäuren, zwischen 1 und 5 % primäre C18-C32-Fettalkohole und zwischen 1 und 3 % C16-C32-Fettsäuren umfasst.

2. Zusammensetzung nach Anspruch 1, **dadurch gekennzeichnet, dass** sie sich in Form einer kosmetischen und/oder dermatologischen, zur Verabreichung auf topischem kutanem Weg geeigneten Zusammensetzung präsentiert, die ein kosmetisch oder pharmazeutisch akzeptables Milieu umfasst.

3. Zusammensetzung nach einem der vorangehenden Ansprüche, **dadurch gekennzeichnet, dass** der hydrolysierte Korkextrakt in einer Menge zwischen 0,0001 Gew.-% und 10 Gew.-% inklusive vorhanden ist, in vorteilhafter Weise in einer Menge zwischen 0,001 Gew.-% und 5 Gew.-% inklusive, und in noch vorteilhafterer Weise in einer Menge zwischen 0,05 Gew.-% und 3 Gew.-% in Bezug zum Gesamtgewicht der entgültigen Zusammensetzung.

4. Zusammensetzung nach einem der vorangehenden Ansprüche, **dadurch gekennzeichnet, dass** der Extrakt zuvor in einem oder mehreren kosmetisch oder pharmazeutisch akzeptablen Lösungsmitteln wie dem Ethanol, dem Propanol oder dem Isopropanol, dem Propylenglycol, dem Butylenglycol, dem Dipropylenglycol, den ethoxylierten oder propoxylierten Diglycolen, oder jedem Gemisch dieser Lösungsmittel gelöst oder dispergiert wird.

5. Zusammensetzung nach einem der vorangehenden Ansprüche, **dadurch gekennzeichnet, dass** der Extrakt zuvor in einem kosmetischen oder pharmazeutischen Vektor wie den Liposomen gelöst oder auf pulvrigen organischen Polymeren, mineralischen Trägern wie den Talken und Bentoniten adsorbiert und im allgemeinen in allen kosmetisch oder pharmazeutisch akzeptablen Vektoren gelöst oder auf ihnen fixiert wird.

6. Zusammensetzung nach einem der vorangehenden Ansprüche, **dadurch gekennzeichnet, dass** sie sich in Form einer wässrigen, hydroalkoholischen oder öligen Lösung präsentiert oder in Form einer Öl-in-Wasser-, Wasser-in-Öl-Emulsion oder multipler Emulsionen oder in Form von Cremes, Suspensionen oder auch von Pulvern, wobei diese Zusammensetzungen mehr oder weniger flüssig oder fest sein und wie eine Creme, eine Lotion, eine Milch, ein Serum, eine Pomade, ein Gel, eine Paste, ein Schaum oder ein Stick aussehen können.

7. Kosmetische Verwendung einer wirksamen Menge mindestens eines hydolysierten Korkextrakts als Wirkstoff, allein oder in Verbindung mit mindestens einem anderen Wirkstoff, wobei der Korkextrakt mehrheitlich Alkohole und Fettsäuren enthält, wobei die Alkohole und Fettsäuren aus der Hydrolyse der Ester des Korkextrakts resultieren, in einer Zusammensetzung als Streckmittel der Haut, um die Haut mechanisch zu glätten.

8. Verwendung nach Anspruch 7 zur Bildung eines Films, der bewirkt, dass sich die obere Hornschicht der Epidermis zusammenzieht und die Haut spannt.

9. Verfahren zur kosmetischen Behandlung der Haut, das dazu bestimmt ist, die Oberfläche der Haut sofort mechanisch zu glätten, das darin besteht, auf die Haut eine nach einem der Ansprüche 1 bis 6 definierte kosmetische Zusammensetzung oder eine Zusammensetzung aufzutragen, die einen nach Anspruch 7 definierten Korkextrakt enthält.
